# EUROPEAN PATENT SPECIFICATION

(11) **EP 4 218 856 B1**
(45) Date of publication and mention of the grant of the patent: **25.11.2026**
(21) Application number: 23170987.4
(22) Date of filing: 29.08.2019
(51) Int. Cl.: A61M 5/142, A61M 5/172, A61M 5/46

(54) **DRUG DELIVERY SYSTEM WITH SENSOR HAVING OPTIMIZED COMMUNICATION AND INFUSION SITE**
ARZNEIMITTELABGABESYSTEM MIT SENSOR MIT OPTIMIERTER KOMMUNIKATIONS- UND INFUSIONSSTELLE
SYSTÈME D'ADMINISTRATION DE MÉDICAMENT AVEC CAPTEUR AYANT UN SITE DE COMMUNICATION ET DE PERFUSION OPTIMISÉ

(30) Priority: 29.08.2018 US 201816116071
(43) Date of publication of application: 02.08.2023
(62) Document number(s) of the earlier application(s) in accordance with Art. 76 EPC: 19194241.6 / 3 616 735
(73) Proprietor: Insulet Corporation, Acton, MA 01720 (US)
(72) Inventor: NAZZARO, David, Groveland (US); D'ARCO, John, Wilmington (US); O'CONNOR, Jason, Acton (US); MCLAUGHLIN, Ian, Groton (US)
(74) Representative: Peterreins Schley

(56) References cited:
- US-A- 5 800 420
- US-A1- 2011 213 306
- US-A1- 2012 184 909

## Description

### TECHNICAL FIELD

Embodiments generally relate to medication delivery. More particularly, embodiments relate to drug delivery systems that rely on associated sensors.

### BACKGROUND

Many conventional drug delivery systems include a drug delivery device and an associated sensor. The sensor can determine and store data related to a user of the drug delivery device. The drug delivery device can then be operated based on the data related to the user. Often, the sensor and the drug delivery device are controlled by a remote device. Conventional sensors generally operate at low power levels to conserve resources. As a result of low transmission power levels, many conventional sensors cannot always relay the stored data to the remote control to ensure efficient operation of the drug delivery device. Further, many conventional drug delivery systems that combine or collocate the sensor and the drug delivery device fail to account for the different durations of use of the sensor and the drug delivery device. As a result, either the sensor is prematurely replaced or the infusion site of the drug delivery device is not spaced far enough away or changed frequently enough in relation to the sensing site of the cannula. Accordingly, there is a need for a drug delivery system that includes a drug delivery device and associated sensor that ensures data from the sensor can be provided to a remote controller device and improves management of the infusion site in view of the sensing site.

US 2012/184909 A1 discloses a drug delivery system according to the state of the art.

### BRIEF DESCRIPTION OF THE DRAWINGS

FIG. 1 illustrates an exemplary drug delivery system.
FIG. 2 illustrates a second view of the drug delivery system depicted in FIG. 1.
FIG. 3 illustrates an overhead view of the drug delivery system depicted in FIG. 1.
FIG. 4 illustrates a cross-sectional side view of the drug delivery system depicted in FIG. 1.
FIG. 5 illustrates an underside view of the drug delivery system depicted in FIG. 1.
FIG. 6 illustrates an exemplary operating environment of the drug delivery system depicted in FIG. 1.
FIG. 7 illustrates an exemplary rotation of infusion sites for a fixed position of a sensing site for the drug delivery system depicted in FIG. 1.
FIG. 8 illustrates an overhead view of a second exemplary drug delivery system.
FIG. 9 illustrates a front view of the drug delivery system depicted in FIG. 8.
FIG. 10 illustrates a side view of the drug delivery system depicted in FIG. 8.
FIG. 11 illustrates an isometric view of the drug delivery system depicted in FIG. 8.
FIG. 12 illustrates a sensor configuration module of the drug delivery system depicted in FIG. 8.
FIG. 13 illustrates a side view of the sensor configuration module depicted in FIG. 12.
FIG. 14 illustrates a third exemplary drug delivery system.

### DETAILED DESCRIPTION

The invention is defined according to claim 1 and dependent claims 2-7 indicating additional embodiments.

This disclosure presents various systems, components, and methods related to a drug delivery system. Each of the systems, components, and methods disclosed herein provides one or more advantages over conventional systems, components, and methods.

According to the invention, disclosed is a drug delivery system having a drug delivery device and an associated sensor. The sensor can be associated with a sensing site on the body of a user. The drug delivery device can be positioned over the sensor in any rotational position and can be associated with an infusion site on the body of the user. The close positioning of the sensor and the drug delivery device allows data from the sensor to be relayed to the drug delivery device and then on to a remote control device. Further, the drug delivery device can be replaced at the end of its duration of use, which is shorter than the duration of use of the sensor, without disturbing the sensor. One or more subsequent drug delivery devices can be used with the sensor while allowing each corresponding infusion site to be changed, thereby providing more efficient operation of the drug delivery system. Other embodiments are disclosed and described.

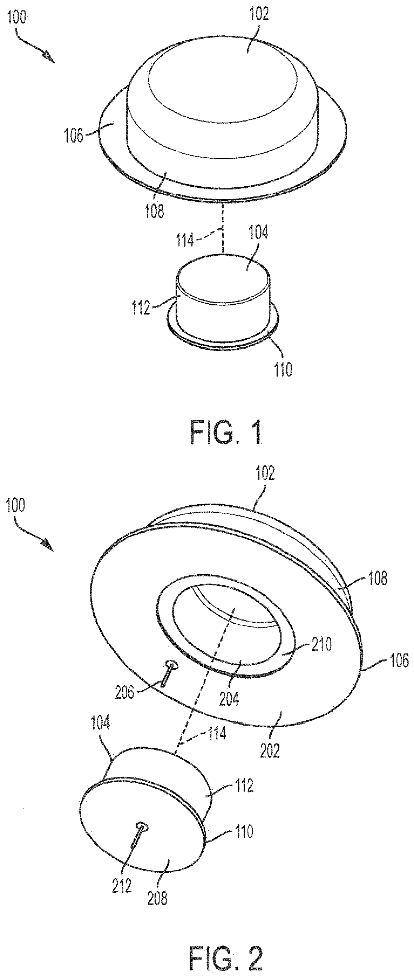

FIG. 1 illustrates an exemplary drug delivery system 100. The drug delivery system 100 includes a drug delivery device 102 and a sensor 104. The drug delivery device 102 can be any type of drug delivery device. The drug delivery device 102 can deliver any type of drug or therapeutic agent to a user. In various embodiments, the drug delivery device 102 can store and deliver insulin to a user and can be considered to be a drug delivery device "pump." FIG. 1 shows a first view of the drug delivery system 100. The sensor 104 can monitor one or more health, biological, biomedical, or medical conditions of a user. The sensor is a continuous glucose monitoring (CGM) sensor.

As shown in FIG. 1, the drug delivery device 102 can include a base component 106 and a housing component 108. The base 106 can be considered a lip component or portion that extends around and beyond the housing 108. The housing 108 can be cylindrically shaped with a circular cross-sectional profile and a tapered top portion but is not so limited. The base 106 can also be circular but is not so limited. In various embodiments, the circular base 106 can have a diameter that is larger than a diameter of the housing 108 such that the base 106 includes an edge or lip portion that extends beyond the outer diameter of the housing 108. The drug delivery device 102 can include all components for storing a liquid drug or other therapeutic agent and providing the stored liquid drug or therapeutic agent to a user of one or more doses.

The CGM sensor 104 can include a base component 110 and a housing component 112. The base 110 can include a lip component or portion that extends around and beyond the housing 112. The housing 112 can be cylindrically shaped with a circular cross-sectional profile but is not so limited. The base 110 can also be circular but is not so limited. In various embodiments, the circular base 110 can have a diameter that is larger than a diameter of the housing 112 such that the base 110 includes an edge or lip portion that extends beyond the outer diameter of the housing 112. The CGM sensor 104 can include all components for monitoring glucose levels of a user. The drug delivery device 102 and the CGM sensor 104 can each have any size, shape, or form factor. In general, the drug delivery device 102 and the CGM sensor 104 can each be made to be small and compact with low profile form factors (e.g., as shown in FIG. 1) to maximize comfort for a user while remaining inconspicuous.

In various embodiments, the drug delivery system 100 can be a wearable drug delivery system. For example, the drug delivery system 100 can be worn directly on the body or skin of a user. In various embodiments, the CGM sensor 1 04 can be positioned within a central opening of the drug delivery device 102 (as indicated by indicator 114). In such embodiments, the base 110 and the housing 112 of the CGM sensor 104 can fit within the central cavity of the drug delivery device 102. The drug delivery device 102 can fit over the CGM sensor 104 such that the bottom surfaces of the drug delivery device 102 and the CGM sensor 104 are aligned and/or level (e.g., coplanar). In this way, the drug delivery system 100 can be easily attached to a user and can rest comfortably on the user.

According to the invention, the arrangement of the drug delivery device 102 and the CGM sensor 104 allows the drug delivery device 102 to be removed and replaced. When replacing the drug delivery device 102, a new drug delivery device 102 can be positioned on top of the CGM sensor 104. The CGM sensor 104 can generally have a duration of use that is longer than the duration of use of the drug delivery device 102. In various embodiments, the CGM sensor 104 can have a first duration of use that can be approximately twelve (12) days. During the duration of use of the CGM sensor 104, a sensing cannula of the CGM sensor 104 can remain fixed in place (e.g., within the body of the user).

In various embodiments, the drug delivery device 102 can have a duration of use that can be approximately three (3) days. During the duration of use of the drug delivery device 102, an infusing cannula of the drug delivery device 102 can remain fixed in place (e.g., within the body of the user). With the drug delivery system 100, the infusion site for a user can be changed each time the drug delivery device 102 is replaced. This is advantageous as an infusion site can become less effective after roughly three (3) days of use.

Accordingly, when a first drug delivery device 102 is removed and replace with a second drug delivery device 102, the infusion site can be changed. The drug delivery device 102 can be replaced several times (e.g., four (4) times) before both the last drug delivery device 102 and the CGM sensor 104 are both removed and replaced together (e.g., at a new site on the body of the user). In this way, the drug delivery system 100 enables a sensing site for glucose monitoring to be maintained for the entire duration of use of the CGM sensor - which increases the effectiveness of the monitoring or sensing site - while providing the flexibility to adjust the infusion site.

FIG. 2 illustrates a second view of the drug delivery system 100. An underside of the drug delivery system 100 is shown in FIG. 2. As shown, the drug delivery device 102 can include a bottom surface 202 and an opening or cavity 204. The opening 204 can be positioned in a center of the bottom surface 202. The opening 204 can be circular but is not so limited. In generally, the opening 204 can be shaped to accommodate the drug delivery device 102 fitting over the CGM sensor 104. The bottom surface 202 can also be circular with an inner diameter (e.g., defining a boundary of the outer portion of the opening and an inner portion of the bottom surface 202) and an outer diameter (e.g., defining a boundary of the outer portion of the bottom surface 202). A cannula 206 (e.g., an infusion cannula) can extend from the bottom surface 202. The cannula 206 can be positioned along any portion of the bottom surface 202 (e.g., offset from a center of the bottom surface 202).

As further shown in FIG. 2, the CGM sensor 104 can include a bottom surface 208. The bottom surface 208 can be circular but is not so limited. An outer portion of the base 110 can fit or be positioned within a recess 210 of the base 106 of the drug delivery device 102. The recess 210 can be positioned around the opening 204. When the CGM sensor 104 is positioned within the opening 204, the outer edge of the bottom surface 208 can reside with the recess 210, thereby allowing the bottom surface 202 and the bottom surface 208 to form a substantially flat surface (e.g., to be aligned or coplanar and/or to form a single continuous surface as felt by the user wearing the drug delivery system 100).

A cannula 212 (e.g., a sensing cannula) can extend from the bottom surface 208. The cannula 212 can be positioned along any portion of the bottom surface 208. In various embodiments, the cannula 212 can extend from a center of the bottom surface 208.

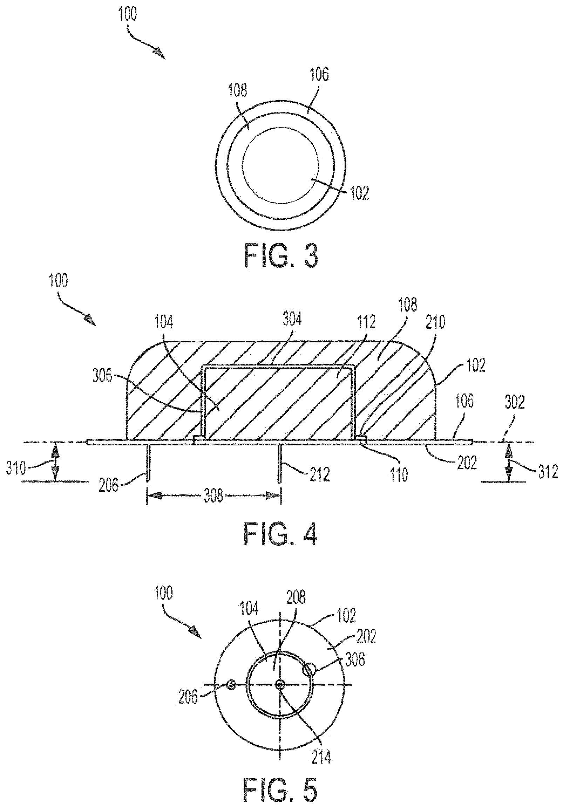

FIG. 3 illustrates an overhead view of the drug delivery system 100. As shown, the drug delivery device 102 covers the CGM sensor 104 such that the CGM sensor 104 is not visible from an overhead view of the drug delivery system 100. An outer portion of the base 106 extends beyond the housing 108. Accordingly, in various embodiments, the width, diameter, and/or perimeter of the base 106 may be larger than the width, diameter, and/or perimeter of the housing 108.

FIG. 4 illustrates a cross-sectional side view of the drug delivery system 100. As shown, the CGM sensor 104 is positioned within the opening 204 of the drug delivery device 102. The CGM 104 can be positioned within the opening 204 such that the bottom surface 208 of the CGM sensor 104 is aligned with the bottom surface 202 of the drug delivery device 102. In various embodiments, the bottom surfaces 202 and 208 can be substantially coplanar - e.g., aligned along an axis 302 as shown in FIG. 4. As shown, in various embodiments, the drug delivery device 102 can cover all outer surfaces of the CGM sensor 104 other than the bottom surface 208 of the CGM sensor 104 - such that, for example, the CGM sensor 104 is encapsulated by the drug delivery device 102 and the body of the user.

FIG. 4 shows a first clearance or distance (or space or spacing) 304 between a top of the housing 112 and an inner upper surface of the housing 108. FIG. 4 shows a second clearance or distance (or space or spacing) 306 between an outer diameter of the housing 112 and a diameter of the opening 204. The first and second clearances 304 and 306 can be of any size. In general, the first and second clearances 304 and 306 can be relatively small (e.g., relative to the overall width of the drug delivery system 100) such that the CGM sensor 104 forms a tight fit inside the opening 204.

As further shown in FIG. 4, an outer portion or lip of the base 110 is positioned within the recess 210 to facilitate the alignment of the bottom surfaces 202 and 208. The cannula 214 is shown spaced a distance 308 from the cannula 206. The distance 308 can be a minimum distance. As the drug delivery device 102 (or a replacement drug delivery device 102) can be positioned over the CGM sensor 104 in any rotational position relative to the CGM sensor 104, the cannula 206 can be positioned the distance 308 away from the cannula 212 according to any rotational position (e.g., along any position along a circle having a radius of distance 308; 360 degrees around the site of the cannula 212).

The cannula 206 can have a depth (e.g., an infusion depth) 310. Similarly, the cannula 212 can have a depth (e.g., a sensing depth) 312. The depths 310 and 312 can be the same depths (as shown in FIG. 4) but are not so limited. The position of the cannula 212 with respect to the body of a user can be considered to be a sensing site. The position of the cannula 206 with respect to the body of the user can be considered to be an infusion site.

In various embodiments, the drug delivery device 102 can be positioned over the CGM sensor 104 in any orientation - for example, the infusion site can be spaced the distance 308 from the sensing site at any angular orientation relative to the sensing site. This allows the infusion site provided by the cannula 206 to be rotated or adjusted around the body of the user while the sensing site remains fixed - for example, when the drug delivery device 102 is removed and replaced with a subsequent or next drug delivery device 102. When removing the drug delivery device 102, the attachment of the CGM sensor 104 can be undisturbed. The next drug delivery device 102 can then be placed over and fitted on top of the CGM sensor 104 in the same manner the original drug delivery device 102 was placed over the CGM sensor 104. Again, this allows the CGM sensor to operate using a sensing site that remains fixed over the duration of use of the CGM sensor while the infusion sites associated with one or more corresponding drug delivery devices 102 can be changed with each new drug delivery device 102.

FIG. 5 illustrates an underside view of the drug delivery system 100 (e.g., a view of the bottom of the drug delivery system 100). As shown, the CGM sensor 104 is positioned within the opening 204 with the clearance 306 separating the CGM sensor 104 from the drug delivery device 102. The cannula 206 is shown separated by the distance 308 from the cannula 212. The bottom surface 202 and/or the bottom surface 208 can include an adhesive to facilitate coupling to the body (e.g., skin) of a user. Any portion of the bottom surface 202 can include an adhesive. Similarly, any portion of the bottom surface 208 can include an adhesive. The adhesive on the bottom surface 208 can be the same adhesive on the bottom surface 202 or can be a different adhesive.

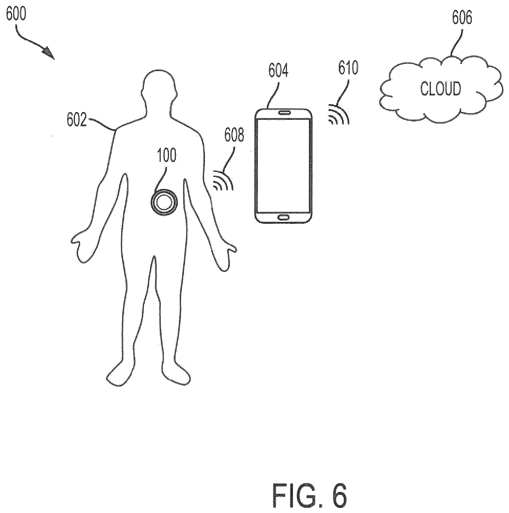

FIG. 6 illustrates an exemplary operating environment 600 of the drug delivery system 100. As shown in FIG. 6, the operating environment can include a user or patient 602, the drug delivery system 100, a controller device 604, and a remote or cloud computing component 606. The user 602 can be a user of the drug delivery system 100. The controller device 604 can be a handheld computing device including a dedicated (e.g., customized) controller or a smartphone for controlling operation of the drug delivery system 100. In various embodiments, the controller device 604 can be a tablet or a desktop computer. In general, the controller device 604 can be any computing device, including a mobile computing device, and can be a commercial off the shelf (COTS) computing device or a customized computing device. The controller device 604 can be used by the user 602 to manage operation of the drug delivery system 100 and to visually observe performance information related to operation of the drug delivery system 100.

The cloud component 606 can represent a remote computing platform. The cloud component 606 can monitor operation of the drug delivery system 100 through connectivity with the controller device 604. The cloud component 606 can store and analyze data related to operation of the drug delivery system 100 (e.g., as determined or adjusted by the controller device 604). The cloud component 606 can be connected to one or more remote computing devices to facilitate viewing or manipulation of any stored operational data. For example, a mobile device of a remote caregiver can receive operational data stored by the cloud component 606 for viewing and/or manipulation.

In various embodiments, a communications link 608 can provide connectivity between the drug delivery system 100 and the controller device 604 and a communications link 610 can provide connectivity between the controller device 604 and the cloud component 606. The communication links 608 and 610 can be bi-direction communication links and can include wired, wireless, optical, and/or infrared communication links. The communication links 608 and 610 can operate according to any known communications standard or protocol over any type of communications medium or link. In various embodiments, a computing device coupled to the cloud component 606 can control operation of the drug delivery system 100 (e.g., by a caregiver during an emergency).

In various embodiments, the CGM sensor 104 can monitor glucose levels and store data indicative of the same. This data can be provided to the drug delivery device 102 to adjust operation of the drug delivery device 102. In various embodiments, data from the CGM sensor 104 can be passed along to the controller device 604 and/or the cloud component 606 by the drug delivery device 102.

To facilitate data sharing between the CGM sensor 104 and the drug delivery device 102, the CGM sensor and the drug delivery device 102 can be connected over a wired, wireless, optical, and or infrared communication link. The communication link coupling the CGM sensor 104 to the drug delivery device 102 can operate according to any known communications standard or protocol over any type of communications medium or link.

In various embodiments, the drug delivery device 102 can communicate with the controller device 604 over Bluetooth Low Energy (BLE). In various embodiments, the CGM sensor 104 can communicate with the drug delivery device 102 over Near-field communication (NFC), a radio-frequency (RF) communication standard, or BLE. In general, the drug delivery device 102 can include more resources (e.g., power resources and/or transmission signal strength) for providing more reliable communications with the controller device 604 compared to the resources (e.g., power resources and/or transmission signal strength) of the CGM sensor 104 for communicating with the drug delivery device 102.

For conventional CGM sensors that communicate with separate (e.g., de-coupled) conventional drug delivery devices, the conventional CGM sensors often are unable to relay stored data to a conventional controller device - e.g., because the communication protocol and/or transmission levels of the conventional CGM sensors are often too low such that the conventional controller devices are often out of range of the conventional CGM sensors. As a result, many conventional CGM sensors cannot provide their monitored glucose data to a conventional controller device such that the data can be used to more efficiently operate a conventional drug delivery device. As a result, often the data collected by the conventional CGM sensor is lost or unused. Alternatively, a user of such a conventional CGM sensor must awkwardly wave her conventional controller device over the conventional CGM sensor repeatedly to ensure reception of the transmitted data. "Picking up" the stored glucose data from the conventional CGM sensor in this manner is often required during inconvenient times for the user, making the process cumbersome and undesirable.

In contrast to such conventional systems and conventional CGM sensors, the drug delivery system 100 provides improved communication capabilities by closely locating (e.g., co-locating) the CGM sensor 104 with the drug delivery device 102. In doing so, even if the CGM sensor 104 uses a traditionally low power method of communication (e.g., a low power wireless standard), the drug delivery device 102 can detect any data transmission from the CGM sensor 104 due to the close proximity of the CGM sensor 104 and the drug delivery device 102. As a result, data from the CGM sensor 104 can be relayed to the controller device 604 (and subsequently on to the cloud component 606) by the drug delivery device 102. In turn, the drug delivery device 102 can be operated more efficiently to better manage the health of the user 602.

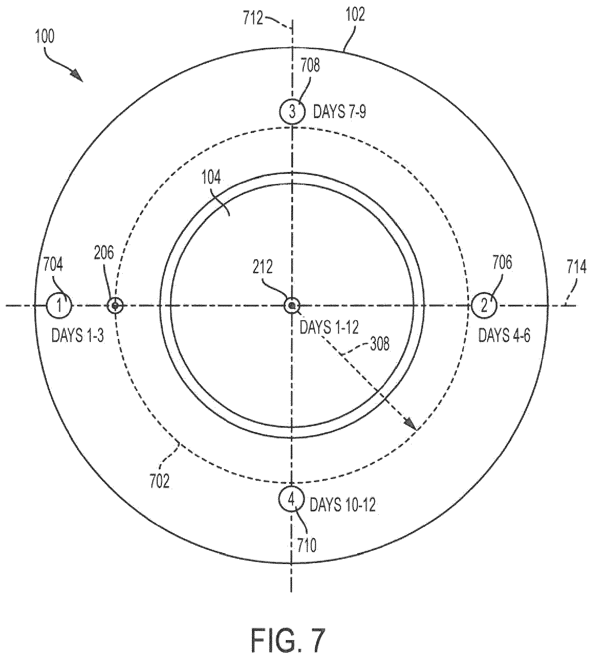

FIG. 7 illustrates an exemplary rotation of infusion sites for a fixed position of a sensing site for the drug delivery system 100 using, for example, multiple replacement drug delivery devices 102. FIG. 7 shows an underside or bottom view of the drug delivery system 100. As shown, an infusion site availability circle 702 surrounds the cannula 212 (or the site of the cannula 212 or sensing site). The circle 702 can be centered about the cannula 212 and can have a radius equal to the distance 308. The circle 702 can represent possible locations of the cannula 206 (or the site of the cannula 206 of the infusion site). Specifically, the cannula 206 can be positioned anywhere along the circle 702. As shown, the cannula 206 is shown positioned along the circle 702.

In various embodiments, the CGM sensor 104 can be worn by a patient for twelve (12) days. Accordingly, the cannula 212 is positioned as shown for the entirety of the twelve (12) days (e.g., the cannula 212 can be located as shown for the entire duration of use of the CGM sensor 104). In various embodiments, the drug delivery device 102 can be used for three (3) days and then replaced with another drug delivery device 102. In various embodiments, the position of the cannula 206 can be changed or adjusted for each new drug delivery device 102. In particular, the position of the cannula 206 can be rotated along the circle 702.

FIG. 7 shows an exemplary rotation of the infusion site provided by rotating the cannula 206 associated with each drug delivery device 102 used with the CGM sensor 104. As shown, site 704 can represent a first or initial site for cannula 206 (e.g., for days 1-3). The first site 704 can be positioned along a first central axis 714. The cannula 212 and the first site 704 can both be positioned along the central axis 714 separated by the distance 308.

Site 706 can represent a second site for the cannula 206 (e.g., for days 4-6). The second site 706 can represent the position of the cannula 206 when a second drug delivery device 102 replaces the first drug delivery device 102 that is associated with the first site 704. The second site 706 can also be positioned along the axis 714. The second site 706 can be positioned 180 degrees away from the first site 704 along the circle 702 relative to the fixed location of the cannula 212.

Site 708 can represent a third site for the cannula 206 (e.g., for days 7-9). The third site 708 can represent the position of the cannula 206 when a third drug delivery device 102 replaces the second drug delivery device 102 that is associated with the second site 706. The third site 708 can be positioned along a second central axis 712. The second central axis 712 can be perpendicular to the first central axis 714. The third site 708 can be positioned 90 degrees away from the second site 706 along the circle 702 relative to the fixed location of the cannula 212.

Site 710 can represent a fourth site for the cannula 206 (e.g., for days 10-12). The fourth site 710 can represent the position of the cannula 206 when a fourth drug delivery device 102 replaces the third drug delivery device 102 that is associated with the third site 708. The fourth site 710 can be positioned along the first axis 714. The fourth site 710 can be positioned 180 degrees away from the third site 708 along the circle 702 relative to the fixed location of the cannula 212.

FIG. 7 shows an exemplary rotation of the infusion site using the cannula 206. In general, any rotation of the cannula 206 along the circle 702 can be used with the position of the infusion site changing with each new drug delivery device 102. After the duration of use of the CGM sensor 104 has expired, a new sensing site for the cannula 212 can be chosen on the user. Further, infusion sites relative to the new sensing site (e.g., spaced a distance 308 away) can then also be chosen relative to the sensing site.

As described herein, a sensor and drug delivery device can have different durations of use. For example, a sensor (e.g., a CGM sensor) can be used for longer periods of time (e.g., coupled to a user) than a drug delivery device (e.g., a wearable drug delivery device such as an insulin pump). In various embodiments, a CGM may have a duration of use of 12 days or longer while a wearable drug delivery device may have a duration of use of 3 days. Accordingly, a drug delivery system incorporating a CGM sensor and a wearable drug delivery device that relies on the CGM sensor for effective operation is to account for the different durations of use in accordance with the techniques disclosed herein.

Further, a sensor (e.g., a CGM sensor) can be sterilized in a first manner that may not be compatible with a wearable drug delivery device. For example, a CGM sensor may be effectively sterilized using gamma radiation. However, for a wearable drug delivery device, which may include electronics, sterilization by gamma radiation may not be possible. Instead, for the wearable drug delivery device, Ethylene Oxide (EO) sterilization may be used, which is not compatible with the CGM sensor. Accordingly, a drug delivery system incorporating a CGM sensor and a wearable drug delivery device that relies on the CGM sensor for effective operation is to account for the different sterilization methods for the two components.

Lastly, many sensors such as a CGM sensor may need to be coupled to a use for a period of time before the site properly provides proper sensor measurements. That is, a CGM sensor may need to be in place for roughly a day or so before measurements made by the CGM sensor can be considered accurate and trustworthy. In contrast, the infusion site associated with a wearable drug delivery device may only be useable for a few days (e.g., at most 3 days) before the site becomes unusable (e.g., due to the user's body resisting the infusion site after a few days). Accordingly, a drug delivery system incorporating a CGM sensor and a wearable drug delivery device that relies on the CGM sensor for effective operation is to account for these different use restrictions.

The disclosed systems, devices, methods, and techniques disclosed herein can account for and accommodate these competing characteristics of a CGM sensor and wearable drug delivery device.

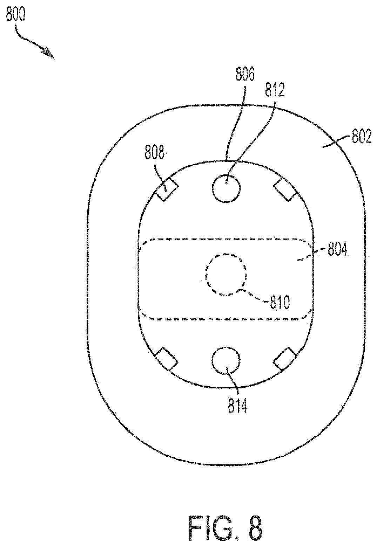

FIG. 8 illustrates an overhead view of a portion of a second exemplary drug delivery system 800. The drug delivery system 800 can be alternative implementation of the drug delivery system 100. FIG. 8 shows an adhesive pad 802 and an incorporated sensor 804 (e.g., a CGM sensor). The CGM sensor 804 can be directly coupled to the adhesive pad 802. This arrangement allows the CGM sensor 804 and adhesive pad to be sterilized according to a first sterilization technique, such as gamma radiation. The cGM sensor 804 can be shown in phantom to show a possible position of the CGM sensor 804 and to reflect that it can be positioned on top of the adhesive pad 802 or can be positioned within an interior of the adhesive pad 802.

An outline 806 indicates where a drug delivery device (e.g., an insulin pump) can be attached to the adhesive pad 802 as described further herein. In various embodiments, the drug delivery device can be separately attached to the adhesive pad 802 having the incorporated CGM sensor 804. In this way, the drug delivery device can be sterilized according to a separate technique more suitable to the drug delivery device and then later coupled to the CGM sensor 804. The drug delivery device can be attached to the adhesive pad by a variety of techniques including using snaps or connectors 808. Any number of snaps and/or connectors 808 can be used.

Indicator 810 shows a position of a sensing site associated with the CGM sensor 804. That is, a sensing needle (or needles) can extend from the underside of the CGM sensor 804 at the site 810. The site 810 is shown in phantom to indicate that the site is position on the underside of the CGM sensor 804 at an opening of the adhesive pad 802. In various embodiments, the adhesive pad 802 can be positioned over an entirety of the underside of the CGM sensor 804 other than at the opening to accommodate the site 810.

In various embodiments, the adhesive pad 802 and the incorporated CGM sensor 804 can be attached to a user. The adhesive pad 802 and the incorporated CGM sensor 804 can be coupled to a user for a first period of time before a drug delivery device is coupled to the adhesive pad 802 and the incorporated CGM sensor 804 (or before a coupled drug delivery device is activated). In this way, the sensing site (or sites) associated with the adhesive pad 802 and the incorporated CGM sensor 804 can operate over the first period of time to reach a steady state (e.g., allowing the sensing sites to become reliable).

In various embodiments, once the CGM sensor 804 has been coupled to the user for a desired period do time, the drug delivery device can be coupled over the CGM sensor 804 and can be coupled to the adhesive pad 802 by the connectors 808. In various embodiments, the connectors 808 can be mechanical and can be, for example, latches or other devices providing a press fit.

In various embodiments, the adhesive pad 802 can include a first infusion site or opening 812 and a second infusion site or opening 814. The drug delivery device when attached to the adhesive pad 802 can use either the firs tor second openings 812 and 814 for deploying an infusion needle or cannula. As the adhesive pad 802 and the CGM sensor 804 can be coupled to the user for a period of time longer than the drug delivery device, an initial drug delivery device can be removed and replaced with a second drug delivery device. The first drug delivery device can use the first opening 812 for the infusion site. The second drug delivery device can use the second opening 814 for the infusion site. In this way, the infusion site can be rotated and changed over the duration of use of the adhesive pad 802 with the incorporated CGM sensor 804.

The openings 812 and 814 can be holes or openings within the adhesive pad 802. The adhesive pad 802 is no limited to the shape depicted. In various embodiments, the adhesive pad 802 can have any shape including round or oval. In various embodiments, the adhesive pad 802 can have multiple openings to facilitate rotation of the infusion site (e.g., more than the two openings 812 and 814). In various embodiments, the drug delivery device can be rotated in any manner with respect to the sensing site opening 810.

In various embodiments, the drug delivery system 800 can be used to initially attach the adhesive pad 802 with the incorporated CGM sensor 804 to a user. The CGM sensor 804 can be configured for operation and can be operated to have one or more sensing sites. After a period of time has elapsed, during which operation of the CGM sensor 804 reaches a steady state (e.g., at a time when measurements made by the CGM sensor 04 are accurate and/or reliable), the drug delivery device can be attached to the adhesive pad 802 (and positioned over top of the CGM sensor 804). The drug delivery device can use on of the two openings 812 and 814 for the first infusion site. After the drug delivery device has been used for a few days, it can be removed/de-coupled from the adhesive pad 802 and replaced with another drug delivery device. The second drug delivery device can use one of the openings 812 and 814 not used by the first drug delivery device. This process can be repeated until the duration of use of the CGM sensor 804 has been reached with each drug delivery device using a different infusion site from the prior drug delivery device.

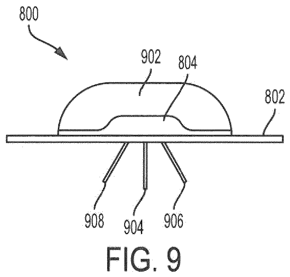

FIG. 9 illustrates a front view of the drug delivery system 800. As shown, the drug delivery system 800 shows the adhesive pad 802 having the incorporated CGM sensor 804 positioned over the adhesive pad 802. Further, a drug delivery device 902 is positioned over the CGM sensor 804. A first cannula 904 is positioned through one of the openings 812 and 814 to provide an infusion site. The first cannula 904 can be coupled to the drug delivery device 902. A second cannula 906 and a third cannula 908 can be coupled to the CGM sensor 804 and can provide first and second sensing sites, respectively. In various embodiments, a single sensing site can be provided and/or more than two sensing sites and cannulas can be used. As shown, the sensing sites provided by the cannulas 906 and 908 are spaced apart from the infusion site provided by the cannula 904.

The drug delivery system 800 and any constituent component thereof can have any size shape, and/or form factor and is not limited to the exemplary shapes shown in FIG. 9 (and/or FIGs. 8 and 10-13).

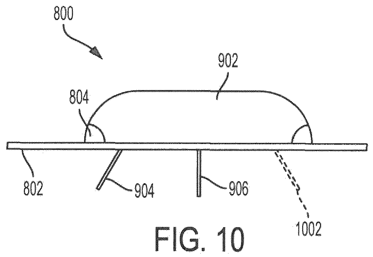

FIG. 10 illustrates a side view of the drug delivery system 800. As shown, the drug delivery device 902 is positioned over the CGM sensor 804 that is incorporated with the adhesive pad 802. The cannula 906 is shown positioned in an approximate middle of the drug delivery system 800. The cannula 904 is shown positioned at a first end of the drug delivery system 800 - for example, using the opening 812. A second cannula 1002 is shown in phantom to represent the location of a second infusion site that can be used through opening 814 when the drug delivery device 902 is replaced.

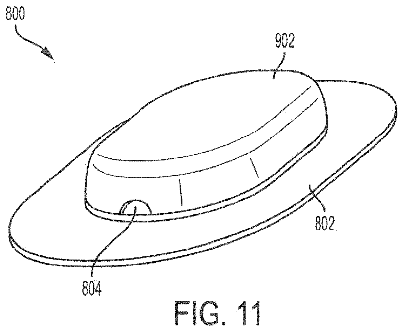

FIG. 11 illustrates an isometric view of the drug delivery system 800. As shown, the drug delivery device 802 is positioned over the CGM sensor 804 incorporated into the adhesive pad 802.

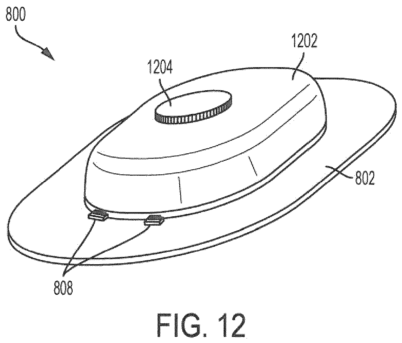

FIG. 12 illustrates a CGM sensor configuration component or module 1202. The CGM sensor configuration module 1202 can be initially coupled to the CGM sensor 804 when provided to the user or can be later attached to the CGM sensor 804 by the user (e.g., using the connectors 808). The CGM sensor configuration module 1202 can be an entirely mechanical based system such that it can be sterilized in the same manner as the adhesive pad 802 and the CGM sensor 804. Alternatively, the CGM sensor configuration module 1202 can include electrical and/or electronical components such that it can undergo a different sterilization technique from the adhesive pad 802 and the CGM sensor 804. Under such a scenario, the CGM sensor configuration module 1202 can be coupled to the adhesive pad 802 and the CGM sensor 804 at a later time.

The CGM sensor configuration module 1202 can be used to configure operation of the CGM sensor 804. After configuring the CGM sensor 804, the CGM sensor configuration module 1202 can be removed and/or detached from the adhesive pad 802 and/or the CGM sensor 804 and replaced with the drug delivery device 902.

In various embodiments, the CGM sensor configuration module 1202 can include a user interface component 1204. The user interface component 1204 can comprise a button and/or a knob. In various embodiments, the user interface component 1204 can be used to activate the CGM sensor 804 - for example, by the user turning a knob and/or pressing a button. In various embodiments, the user interface component 1204 can be used to set a number of sensing sites to be used (e.g., a number of sensing cannulas to deploy), set a depth of sensing (e.g., set a depth for each sensing cannula), and/or to trigger release of the cannulas. Once the one or more sensing cannulas (e.g., cannulas 906 and 908) are deployed, the CGM sensor 804 can begin operation.

After the CGM sensor configuration module 1202 has been used to configure operation of the CGM sensor 804, the CGM sensor configuration module 1202 can be removed from the adhesive pad 802. After the CGM sensor 804 has been operating for an appropriate amount of time, the drug delivery device 902 can be coupled to the adhesive pad 802 and positioned over the CGM sensor 804. The drug delivery device 902 can be coupled in a manner to enable glucose measurements or other data collected or derived by the CGM sensor 804 to be passed to the drug delivery device 902 for use in controlling operation of the drug delivery device 902 (e.g., adjusting a dose of a medicine provided to the user).

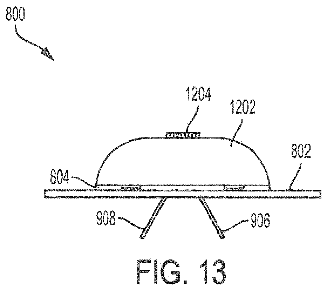

FIG. 13 illustrates a side view of the CGM sensor configuration module 1202 depicted in FIG. 12. As shown in FIG. 13, the CGM sensor configuration module 1202 is positioned over the CGM sensor 804. The cannulas 906 and 908 have been deployed indicating the CGM sensor 804 has been configured for operation. Accordingly, the CGM sensor configuration module 1202 can be removed and replaced with the drug delivery device 902.

In various embodiments, the adhesive pad 802 can have any number of holes positioned on any portion of the adhesive pad 802. In various embodiments, the adhesive pad 802 can have four holes positioned a same distance from a center of a bottom surface of the adhesive pad 802. The four holes can be positioned along a circle that surrounds the center of the bottom surface of the adhesive pad 802, with the center representing a position from which the one or more sensing cannulas of the CGM sensor 804 extend.

In various embodiments, the CGM sensor 804 can be incorporated into the adhesive pad 802 to form a single combined component. In various embodiments, the CGM sensor 804 can only include mechanical components and does not contain any electrical components. In various embodiments, the drug delivery device 902 can include mechanical and/or electrical components. In various embodiments, the CGM sensor configuration module 1202 can include mechanical and/or electrical components. This can allow the drug delivery device 902 and the CGM sensor configuration module 1202 to undergo a sterilization process that is different from a sterilization process that the adhesive pad 802 and incorporated CGM sensor 804 can undergo. In various embodiments, the CGM sensor configuration module 1202 can include only mechanical components, allow the CGM sensor configuration module 1202, the adhesive pad 802, and the incorporated CGM sensor 804 to all undergo the same sterilization process and to be provided to a user connected together as one assembly. Then, once the user configures operation of the CGM sensor 104, the user can snap off the CGM sensor configuration module 1202 and replace it with the drug delivery device 902.

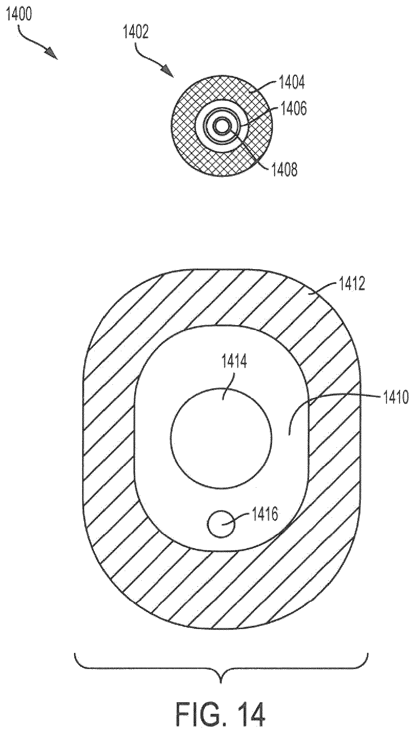

FIG. 14 illustrates a third exemplary drug delivery system 1400. The drug delivery system 1400 can be alternative implementation of the drug delivery system 100. As shown in FIG. 14, the drug delivery system 1400 can include a sensor 1402. The sensor 1402 can be a CGM sensor. FIG. 14 shows a view of the bottom or underside of the sensor 1402. In various embodiments, the CGM sensor 1402 can include an adhesive pad 1404, a first electrode 1406, and a second electrode 1408. The CGM sensor 1402 can be circularly-shaped but is not so limited.

In various embodiments, the adhesive pad 1404 can be positioned toward a periphery or outer portion of the bottom surface of the CGM sensor 1402. The adhesive pad 1404 can be arranged in a first circle. The first electrode 1406 can be positioned toward a middle portion of the bottom surface of the CGM sensor 1402. The first electrode1406 can be arranged in a second circle (e.g., concentric with the first circle of the adhesive pad 1404 and smaller). The second electrode 1408 can be positioned toward a central portion of the bottom surface of the CGM sensor 1402. The second electrode 1408 can be arranged in a third circle (e.g., concentric with the first circle of the adhesive pad 1404 and second circle of the first electrode 1406 and smaller).

In various embodiments, the CGM sensor 1402 can include a sensing cannula. The sensing cannula can extend from any portion of the bottom surface of the CGM sensor 1402 (e.g., from a center of the bottom surface). In various embodiments, the CGM sensor 1402 can be coupled to a user for multiple days - for example, for a period of time of 10 days or more.

FIG. 14 further shows a drug delivery device 1410. The drug delivery device 1410 can be an alternative implementation of the drug delivery device 102. FIG. 14 can show a top view of the drug delivery device 1410. The drug delivery device 1410 can include an adhesive pad 1412. The adhesive pad 1412 can be coupled to a bottom surface of the drug delivery device 1410. The drug delivery device 1410 can be attached to a user by the adhesive pad 1412 and intended to be used for a duration of time that is less than a duration of use of the CGM sensor 1402. For example, the duration of use of the drug delivery device 1410 can be approximately 3 to 4 days, after which the drug delivery device 1410 can be removed from the user (e.g., replaced by a next or subsequent drug delivery device 1410).

The drug delivery device 1410 can include an opening 1414. The CGM sensor 1402 can be positioned into the opening 1414. When positioned in the opening 1414, the CGM sensor 1402 can be coupled to the body of the user (e.g., the adhesive pad 1404 and the first and second electrodes 1406 and 1408 can contact the body of the user). Further, the CGM sensor 1402 can be electrically coupled to the drug delivery device 1410 when positioned in the opening 1414 to allow data from the CGM sensor 1402 to be provided to the drug delivery device 1410.

The drug delivery device 1410 can include an infusion cannula. Indicator 1416 shows a position or site of the infusion cannula (in phantom to indicate the site 1416 extends from the bottom surface of the drug delivery device 1410). In various embodiments, the CGM sensor 1402 can first be coupled to the body of a user. The drug delivery device 1410 can then be positioned over the CGM sensor 1402 such that the CGM sensor 1402 is positioned within the opening 1414. The drug delivery device 1410 can be positioned in any orientation relative to the CGM sensor 1402 such that the infusion site 1416 can be at any rotational position from a center of the CGM sensor 1402.

When the first drug delivery device 1410 has been used for its duration of use, it can be detached from the user without disrupting the position of the CGM sensor 1402. A second or subsequent drug delivery device 1410 can then be positioned over the CGM sensor 1402 and attached to the user. The second drug delivery device 1410 can be rotated relative to the former position of the first drug delivery device 1410 such that the infusion sites 1412 differ (e.g., vary by at least 90 degrees relative to a center of the CGM sensor 1402). In this way, the drug delivery system 1400 enables an unlimited number of orientations of the drug delivery device 1410 relative to the CGM sensor 1402 and therefore an unlimited number of possible infusion sites 1416 that can be changed over the course of use of the CGM sensor 1402 as multiple drug delivery device 1410 are used with the same CGM sensor 1402.

In various embodiments, the CGM sensor 1402 can include electronic components. In various embodiments, the drug delivery device 1410 can include only mechanical components. In various embodiments, the CGM sensor 1402 can include a controller component to control operation of the drug delivery device 1410.

Each of the drug delivery devices described herein can be a wearable or on-body drug delivery device or pump, such as an OmniPod (Insulet Corporation, Billerica, MA, USA) device and/or any of the drug delivery devices described in U.S. Patent Nos. 7,303,549; 7,144,384; 7,137,964; 6,960,192; 6,740,059; 6,699,218; 9,402,950; 7,771,412; 7,029,455; 6,740,05; and 6,656,159. As such, each of the drug delivery devices disclosed herein can include one or more reservoirs or chambers configured to store a liquid drug and a drug delivery mechanism or component for extracting (e.g., pumping) the liquid drug out of the reservoir for delivery to a user. Further, each of the drug delivery devices disclosed herein can include one or more infusion cannulas for provided the stored liquid drug to the user over one or more doses and/or based on data provided by any of the sensors disclosed herein. In each of the disclosed embodiments, the sensor can be coupled to the drug delivery device in any manner (e.g., over a wired or wireless link) to allow the bidirectional flow of any data (e.g., control data, user data, etc.).

The invention is defined according to the appended claims and is not limited to the above embodiments, but rather, additions and modifications to what was expressly described herein are also included within the scope of the invention. Moreover, it is to be understood that the features of the various embodiments described herein were not mutually exclusive and can exist in various combinations. In fact, variations, modifications, and other implementations of what was described herein will occur to those of ordinary skill in the art without departing from the scope of the invention.

## Claims

1. A drug delivery system (800), comprising
an adhesive pad (802) having a plurality of openings, wherein the plurality of openings comprises at least a first opening (812) and a second opening (814) configured to provide access to a body of a user, a bottom surface of the adhesive pad configured to be coupled to the body of the user;
a continuous glucose monitor (CGM) sensor (804) attached to the adhesive pad (802), the CGM sensor (804) having one or more sensing cannulas extendable from a bottom surface of the adhesive pad (802);
a CGM sensor configuration module (1202) configured to be releasably positioned over the CGM sensor (804), the CGM sensor configuration module having a user interface component comprising a button or a knob for triggering deployment of the one or more sensing cannulas of the CGM sensor (804) when the CGM sensor configuration module (1202) is positioned over the CGM sensor (804), the CGM sensor configuration module further configured to be removed after deployment of the one or more sensing cannulas; and
a drug delivery device (902) configured to be positioned over the CGM sensor (804), the drug delivery device (902) having an infusion cannula configured to extend through the first opening of the adhesive pad (802) to reach the body of the user, the drug delivery device (902) configured to be removed from its position over the CGM sensor (804) after a first period of time without disrupting the position of the CGM sensor (804) and to be replaced by a second drug delivery device (902) configured to be positioned over the CGM sensor (804), the second drug delivery device (902) having an infusion cannula configured to extend through the second opening of the adhesive pad (802) to reach the body of the user;
wherein the adhesive pad (802) and the CGM sensor (804) are configured to be coupled to the body of the user for a period of time longer than the first period of time;
**characterised in that**
the first opening and second opening are positioned a same distance from the one or more sensing cannulas.

2. The drug delivery system (800) of claim 1, wherein the CGM configuration module is an entirely mechanical based system.

3. The drug delivery system (800) according to claim 1 or 2, wherein CGM sensor configuration module (1202) includes electrical components.

4. The drug delivery system (800) according to any one of the preceding claims, wherein the first opening (812) is used with the drug delivery device (902) until an end of the first period of time, and the second opening (814) is used by the second drug delivery device.

5. The drug delivery system (800) according to any one of the preceding claims, wherein the plurality of openings in the adhesive pad (802) includes four holes positioned a same distance from a center of a bottom surface of the adhesive pad (802), and
the four holes are positioned along a circle that surrounds the center of the bottom surface of the adhesive pad (802), with the center representing a position from which the one or more sensing cannulas of the CGM sensor (804) extend.

6. The drug delivery system (800) according to any one of the preceding claims, wherein the drug delivery device (902) and the CGM sensor configuration module (1202) are configured to undergo a sterilization process that is different from a sterilization process that the adhesive pad (802) and CGM sensor (804) undergo.

7. The drug delivery system (800) according to any one of the preceding claims, wherein the drug delivery device (902) is configured to replace the CGM sensor configuration module (1202) after removal and/or detachment of CGM sensor configuration module (1202) from the CGM sensor (804).

## Patentansprüche

1. Arzneimittelabgabesystem (800), umfassend
ein Klebepad (802) mit einer Vielzahl von Öffnungen, wobei die Vielzahl von Öffnungen zumindest eine erste Öffnung (812) und eine zweite Öffnung (814) umfasst, die dazu ausgelegt sind, Zugang zu einem Körper eines Anwenders zu ermöglichen, wobei eine Unterseite des Klebepads dazu ausgelegt ist, am Körper des Anwenders befestigt zu werden; einen an dem Klebepad (802) angebrachten Sensor (804) für kontinuierliche Glukoseüberwachung (CGM), wobei der CGM-Sensor (804) eine oder mehrere Messkanülen aufweist, die aus einer Unterseite des Klebepads (802) ausfahrbar sind;
ein CGM-Sensor-Konfigurationsmodul (1202), das dazu ausgelegt ist, lösbar über dem CGM-Sensor (804) positioniert zu werden, wobei das CGM-Sensor-Konfigurationsmodul eine Benutzerschnittstellenkomponente aufweist, die eine Taste oder einen Knopf umfasst, um das Herausfahren der einen oder der mehreren Messkanülen des CGM-Sensors (804) auszulösen, wenn das CGM-Sensor-Konfigurationsmodul (1202) über dem CGM-Sensor (804) positioniert ist, und wobei das CGM-Sensor-Konfigurationsmodul ferner dazu ausgelegt ist, nach dem Herausfahren der einen oder der mehreren Messkanülen entfernt zu werden; und eine Arzneimittelabgabevorrichtung (902), die dazu ausgelegt ist, über dem CGM-Sensor (804) positioniert zu werden, wobei die Arzneimittelabgabevorrichtung (902) eine Infusionskanüle aufweist, die dazu ausgelegt ist, sich durch die erste Öffnung des Klebepads (802) zu erstrecken, um den Körper des Anwenders zu erreichen, wobei die Arzneimittelabgabevorrichtung (902) dazu ausgelegt ist, nach einer ersten Zeitspanne aus ihrer Position über dem CGM-Sensor (804) entfernt zu werden, ohne die Position des CGM-Sensors (804) zu beeinträchtigen, und durch eine zweite Arzneimittelabgabevorrichtung (902) ersetzt zu werden, die dazu ausgelegt ist, über dem CGM-Sensor (804) positioniert zu werden, wobei die zweite Arzneimittelabgabevorrichtung (902) eine Infusionskanüle aufweist, die dazu ausgelegt ist, sich durch die zweite Öffnung des Klebepads (802) zu erstrecken, um den Körper des Anwenders zu erreichen;
wobei das Klebepad (802) und der CGM-Sensor (804) dazu ausgelegt sind, für eine längere Zeitspanne als die erste Zeitspanne am Körper des Anwenders befestigt zu werden; **dadurch gekennzeichnet, dass** die erste Öffnung und die zweite Öffnung in gleichem Abstand zu der einen oder den mehreren Messkanülen angeordnet sind.

2. Arzneimittelabgabesystem (800) nach Anspruch 1, wobei das CGM-Konfigurationsmodul ein vollständig mechanisch basiertes System ist.

3. Arzneimittelabgabesystem (800) nach Anspruch 1 oder 2, wobei das CGM-Sensor-Konfigurationsmodul (1202) elektrische Komponenten umfasst.

4. Arzneimittelabgabesystem (800) nach einem der vorhergehenden Ansprüche, wobei die erste Öffnung (812) bis zu einem Ende der ersten Zeitspanne mit der Arzneimittelabgabevorrichtung (902) verwendet wird und die zweite Öffnung (814) von der zweiten Arzneimittelabgabevorrichtung verwendet wird.

5. Arzneimittelabgabesystem (800) nach einem der vorhergehenden Ansprüche, wobei die Vielzahl von Öffnungen im Klebepad (802) vier Löcher umfasst, die in gleichem Abstand von einem Mittelpunkt einer Unterseite des Klebepads (802) positioniert sind, und
die vier Löcher entlang eines Kreises positioniert sind, der den Mittelpunkt der Unterseite des Klebepads (802) umgibt, wobei der Mittelpunkt eine Position darstellt, von der aus sich die eine oder die mehreren Messkanülen des CGM-Sensors (804) erstrecken.

6. Arzneimittelabgabesystem (800) nach einem der vorhergehenden Ansprüche, wobei die Arzneimittelabgabevorrichtung (902) und das CGM-Sensor-Konfigurationsmodul (1202) dazu ausgelegt sind, einem Sterilisationsverfahren unterzogen zu werden, das sich von einem Sterilisationsverfahren unterscheidet, dem das Klebepad (802) und der CGM-Sensor (804) unterzogen werden.

7. Arzneimittelabgabesystem (800) nach einem der vorhergehenden Ansprüche, wobei die Arzneimittelabgabevorrichtung (902) dazu ausgelegt ist, das CGM-Sensor-Konfigurationsmodul (1202) nach dem Entfernen und/oder Ablösen des CGM-Sensor-Konfigurationsmoduls (1202) vom CGM-Sensor (804) zu ersetzen.

## Revendications

1. Système d'administration de médicament (800), comprenant :
un tampon adhésif (802) présentant une pluralité d'ouvertures, la pluralité d'ouvertures comprenant au moins une première ouverture (812) et une seconde ouverture (814) configurées pour fournir l'accès au corps d'un utilisateur, une surface inférieure du tampon adhésif étant configurée pour être couplée au corps de l'utilisateur ;
un capteur de surveillance du glucose en continu (CGM) (804) fixé au tampon adhésif (802), le capteur CGM (804) ayant une ou plusieurs canules de détection pouvant s'étendre à partir d'une surface inférieure du tampon adhésif (802) ;
un module de configuration de capteur CGM (1202) configuré pour être positionné de manière amovible au-dessus du capteur CGM (804), le module de configuration de capteur CGM ayant un composant d'interface utilisateur comportant un bouton ou un bouton pour déclencher le déploiement de la ou des canules de détection du capteur CGM (804) lorsque le module de configuration de capteur CGM (1202) est positionné au-dessus du capteur CGM (804), le module de configuration de capteur CGM étant en outre configuré pour être retiré après le déploiement de la ou des canules de détection ; et
un dispositif d'administration de médicament (902) configuré pour être positionné au-dessus du capteur CGM (804), le dispositif d'administration de médicament (902) ayant une canule de perfusion configurée pour s'étendre à travers la première ouverture du tampon adhésif (802) pour atteindre le corps de l'utilisateur, le dispositif d'administration de médicament (902) étant configuré pour être retiré de sa position au-dessus du capteur CGM (804) après une première période de temps sans perturber la position du capteur CGM (804) et pour être remplacé par un second dispositif d'administration de médicament (902) configuré pour être positionné au-dessus du capteur CGM (804), le second dispositif d'administration de médicament (902) comportant une canule de perfusion configurée pour s'étendre à travers la seconde ouverture du tampon adhésif (802) pour atteindre le corps de l'utilisateur ;
le tampon adhésif (802) et le capteur CGM (804) étant configurés pour être couplés au corps de l'utilisateur pendant une période de temps plus longue que la première période de temps ; **caractérisé en ce que** la première ouverture et la seconde ouverture sont positionnées à une même distance de la ou des canules de détection.

2. Système d'administration de médicament (800) selon la revendication 1, dans lequel le module de configuration de capteur CGM est un système entièrement mécanique.

3. Système d'administration de médicament (800) selon la revendication 1 ou 2, dans lequel le module de configuration de capteur CGM (1202) comprend des composants électriques.

4. Système d'administration de médicament (800) selon l'une quelconque des revendications précédentes, dans lequel la première ouverture (812) est utilisée avec le dispositif d'administration de médicament (902) jusqu'à une fin de la première période de temps, et la seconde ouverture (814) est utilisée par le second dispositif d'administration de médicament.

5. Système d'administration de médicament (800) selon l'une quelconque des revendications précédentes, dans lequel la pluralité d'ouvertures dans le tampon adhésif (802) comprend quatre trous positionnés à une même distance d'un centre d'une surface inférieure du tampon adhésif (802), et
les quatre trous sont positionnés le long d'un cercle qui entoure le centre de la surface inférieure du tampon adhésif (802), le centre représentant une position à partir de laquelle s'étendent la ou les canules de détection du capteur CGM (804).

6. Système d'administration de médicament (800) selon l'une quelconque des revendications précédentes, dans lequel le dispositif d'administration de médicament (902) et le module de configuration de capteur CGM (1202) sont configurés pour subir un processus de stérilisation qui est différent d'un processus de stérilisation que subissent le tampon adhésif (802) et le capteur CGM (804).

7. Système d'administration de médicament (800) selon l'une quelconque des revendications précédentes, dans lequel le dispositif d'administration de médicament (902) est configuré pour remplacer le module de configuration de capteur CGM (1202) après le retrait et/ou le détachement du module de configuration de capteur CGM (1202) du capteur CGM (804).
